# EUROPEAN PATENT SPECIFICATION

(11) **EP 3 750 529 B1**
(45) Date of publication and mention of the grant of the patent: **30.10.2024**
(21) Application number: 19751781.6
(22) Date of filing: 07.02.2019
(51) Int. Cl.: A61K 31/165, A61K 31/16, A23L 33/10, A61P 21/00, A61P 25/04

(54) **COMPOSITION COMPRISING TISOLAGILINE MESYLATE FOR USE IN THE PREVENTION AND TREATMENT OF SPINAL CORD INJURY**
ZUSAMMENSETZUNG ENTHALTEND TISOLAGILIN METHANSULFONAT ZUR VORBEUGUNG UND BEHANDLUNG VON RÜCKENMARKSLÄSIONEN
COMPOSITION COMPRENANT DU MÉSYLATE DE TISOLAGILINE DESTINÉE À ÊTRE UTILISÉE DANS LA PRÉVENTION ET LE TRAITEMENT D'UNE LÉSION DE LA MOELLE ÉPINIÈRE

(30) Priority: 07.02.2018 KR 20180015296
(43) Date of publication of application: 16.12.2020
(73) Proprietor: Neurobiogen Co., Ltd., Seongnam-si Gyeonggi-do 13201 (KR)
(72) Inventor: PARK, Ki Duk, Seoul 02792 (KR); LEE, Changjoon, Seoul 02792 (KR); PAE, Ae Nim, Seoul 02792 (KR); OH, Soo-Jin, Seoul 02792 (KR); LIM, Sang Min, Seoul 02792 (KR); PARK, Jong Hyun, Seoul 02792 (KR); LEE, Jungmoo, Seoul 02792 (KR); HA, Yoon, Seoul 03722 (KR); LEE, Hye Yeong, Seoul 03786 (KR)
(74) Representative: Cabinet Chaillot
(86) International application number: PCT/KR2019/001497
(87) International publication number: WO 2019/156465

(56) References cited:
- WO-A1-2016/052928
- KR-A- 20090 010 186
- KR-A- 20090 018 817
- KR-A- 20150 080 428
- KR-A- 20160 039 817
- PARK, K. D.: "Development of a novel inhibitor of aberrant gaba synthesis in reactive astrocytes in alzheimer' s disease", NEURODEGENER DISEASE, vol. 12, 2017, pages 893, XP029769442

## Description

### [Technical Field]

The present invention relates to a composition for use in the prevention or treatment of spinal cord injury and a food composition for use in the prevention or alleviation of spinal cord injury, each containing (S)-2-(((4'-trifluoromethylbiphenyl-4-yl)methyl)amino)propanamide methanesulfonate.

### [Background Art]

Damage to the spinal nerve due to trauma or the like causes paralysis of human functions. Spinal nerve tissue has a simpler structure than the brain, but has a problem in that nerve regeneration is considerably difficult. The pathological phenomena that occur after spinal cord injury are classified, based on passage of time, into two phases, that is, primary damage and secondary damage. Primary damage is a phenomenon occurring within several minutes immediately after the damage, resulting in necrosis of the cells at the wound site. In this stage, pharmacological treatment is almost impossible due to rapid destruction of the cells. Secondary damage, which follows primary damage, progresses slowly over several hours to several days, resulting in degeneration of cells around the wound site as well as slow cell death due to apoptosis in the surrounding nerve cells and oligodendrocytes. Cell death continuously progresses around the wound, and eventually, the injured site inside the spinal cord gradually increases in size. In addition, degeneration of axons, which act as pathways for the movement of nerve signals, and of myelin sheaths, which help the functions of the axons, causes formation of empty spaces, such as cavities, making it impossible to transfer nerve signals and resulting in permanent loss of functions.

Over the past decade, research has been conducted to suppress or alleviate permanent functional paralysis caused by spinal cord injury based on studies to investigate the causes of pathological actions after trauma on spinal nerves and regeneration of the spinal nerves. In particular, it is difficult to respond to the initial mechanism of spinal cord injury using pharmacological treatment because the spinal cord injury rapidly progresses initially. Thus, a pharmacological strategy to respond to secondary mechanisms is an important part of the development of therapeutics. In this regard, various potential pharmacological treatments have been attempted to date, including steroids, antioxidants, glutamate receptor inhibitors, ion channel inhibitors, gangliosides, antibodies to axon regeneration inhibitors, anti-inflammatory agents, and neurotrophic factors. Among them, only methylprednisolone is currently used as a therapeutic agent administered after spinal cord injury. However, methylprednisolone has many problems such as insufficiently proven therapeutic effects and side effects upon administration of excessive dosages thereof. Thus, there is an urgent need for the development of a new therapeutic agent.

WO 2016/052928 A1 refers to (S)-2-(((4'-trifluoromethylbiphenyl-4-yl)methyl)amino)propanamide methanesulfonate (KDS 2010) for preventing or treating neurodegenerative disorder.
Park, K. D. "Development of a novel inhibitor of aberrant gaga synthesis in reactive astrocytes in Alzheimer's disease", Neurodegener Disease, vol. 12, 2017, page 893 discloses the use of KDS2010 for treating Alzheimer's disease.
KR 2016 0039817 A refers to alpha-aminoamide derivative compound for treating Parkinson's disease, Alzheimer's disease, epilepsy, and depression.
KR 2009 0018817 A refers to the use of alpha-aminoamides for imroving cognitive function and treating cognitive impairment.
KR 2015 0080428 A discloses the use of amide derivatives for inhibiting pain caused by peripheral nerve damage, central nervous system damage and inflammation as well and have an excellent effect of inhibiting MMP-2 and MMP-9 which are activated after spinal cord injury.
KR 2009 0010186 A discloses the use of salicylic acid derivative compounds to treat or prevent degenerative brain neuronal disease among which degenerative spinal cord injury.

### [Disclosure]

### [Technical Problem]

As a result of extended efforts to solve the problems with the prior art, the present inventors found that the compound (S)-2-(((4'-trifluoromethylbiphenyl-4-yl)methyl)amino)propanamide methanesulfonate of the present invention has an effect of restoring lost nerve function caused by spinal cord injury, relieving pain and facilitating recovery of injured spinal cord tissue and is thus useful for the prevention and treatment of spinal cord injury. Based on this finding, the present invention has been completed.

### [Technical Solution]

In accordance with one aspect of the present invention, the above and other objects can be accomplished by the provision of a pharmaceutical composition for use in the prevention or treatment of spinal cord injury, containing a compound represented by the following Formula 1 as an active ingredient.

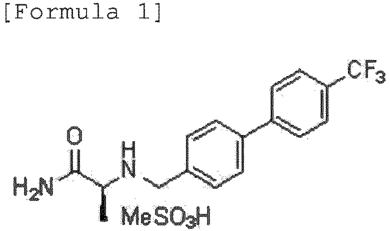

In another aspect of the present invention, provided is a food composition for use in the prevention or alleviation of spinal cord injury containing the compound as an active ingredient.

### [Advantageous Effects]

The composition containing the compound of Formula 1 of the present invention has excellent effects in preventing, alleviating and treating spinal cord injury by restoring deterioration in nerve functions caused by spinal cord injury, relieving pain, and facilitating recovery of injured spinal cord tissue.

### [Description of Drawings]

FIG. 1 is a diagram showing the overall experimental period and the period of administration of the drug (KDS2010);
FIG. 2A is a graph identifying the effect of facilitating the recovery of nerve functions when conducting a behavior test using a Basso-Beattie-Bresnahan (BBB) locomotor rating system after administration of KDS2010 to spinal cord-injured rats and normal rats;
FIG. 2B is a graph identifying the effect of relieving pain response when conducting pain response evaluation using a dynamic plantar test after administration of KDS2010 to spinal cord-injured rats and normal rats;
FIG. 3 includes images showing the degree of myelination of tissues through EC staining of the injured area of tissue obtained after administration of KDS2010 to spinal cord-injured rats and normal rats, and graphs comparing the total tissue area, the total myelinated area, and the proportion (%) of the total myelinated area to the total tissue area;
FIGS. 4A to 4D show the number of myelinated exons, the number of cavities and the g-ratio value, measured by toluidine blue (TB) staining and TEM imaging, wherein Sham is an animal model not administered with a spinal-cord-injury-inducing substance, and V is a control group for KDS;
FIGS. 5A to 5B show the number of newly formed nerve cells measured as a result of immunofluorescence staining; and
FIG. 6 shows the number of inflammatory cells measured through immunofluorescence staining.

### [Best Mode]

Hereinafter, the present invention will be described in detail. Meanwhile, each description and embodiment disclosed in the present invention can be applied to other descriptions and embodiments. That is, all combinations of the various elements disclosed in the present invention fall within the scope of the present invention. In addition, the scope of the present invention is not limited by the specific description given below.

In addition, those skilled in the art to which the present invention pertains can conceive or recognize a number of equivalents to certain embodiments disclosed in the present invention merely through ordinary experimentation. Furthermore, these equivalents should be construed as falling within the scope of the present invention.

In the present invention, the compound represented by Formula 1 is (S)-2-(((4'-trifluoromethyl biphenyl-4-yl)methyl)amino)propanamide methanesulfonate. In the present invention, the compound may be referred to as "KDS2010".

Although the study of the specific pharmacological activity of the compound is still underway, it has not been revealed whether or not the compound has direct effects of preventing and treating spinal cord injury.

In the present invention, there is no particular limitation as to the method for obtaining (S)-2-(((4'-trifluoromethyl biphenyl-4-yl)methyl)amino)propanamide methanesulfonate, and the compound can be chemically synthesized by a method known in the art or may be selected from commercially available substances.

The (S)-2-(((4'-trifluoromethyl biphenyl-4-yl)methyl)amino)propanamide methanesulfonate of the present invention may be present in a solvated form or an unsolvated form. The (S)-2-(((4'-trifluoromethyl biphenyl-4-yl)methyl)amino)propanamide methanesulfonate of the present invention may be present in a crystalline or amorphous form and all of these physical forms fall within the scope of the present invention.

As used herein, the term "spinal cord injury" means a disorder associated with spinal cord injury caused by a trauma such as a traffic or falling accident, which causes loss of movement and thus loss of sensation due to paralysis of motor nerves beneath the injured site, and abnormalities in regulation of bladder and intestinal movement regulated by the autonomic nervous system. In general, spinal cord injury is classified into complete spinal cord injury and incomplete spinal cord injury depending on the degree of injury. "Complete spinal cord injury" refers to a spinal cord injury which is a state of a completely transversely cut spinal cord, and results in loss of motor and sensory ability due to loss of all spinal cord functions below the injured spinal cord site. Incomplete spinal cord injury refers to a spinal cord injury which preserves some sensation or motor function below the injured site and results from dislocated bones, bruising due to edema in soft tissue or spinal canal, or partial severing of the spinal cord. In general, symptoms vary depending on the location of the injured area, and injury to the cervical region has symptoms such as reduced blood pressure, pulse, body temperature and respiratory rate, and may also cause respiratory distress. Injury to thoracic and lumbar regions leads to loss of motor and sensory functions of the trunk and limbs, and loss of bladder, colon and sexual functions.

The pharmaceutical composition of the present invention can facilitate nerve function recovery, relieve pain due to spinal cord injury, or facilitate spinal cord tissue recovery.

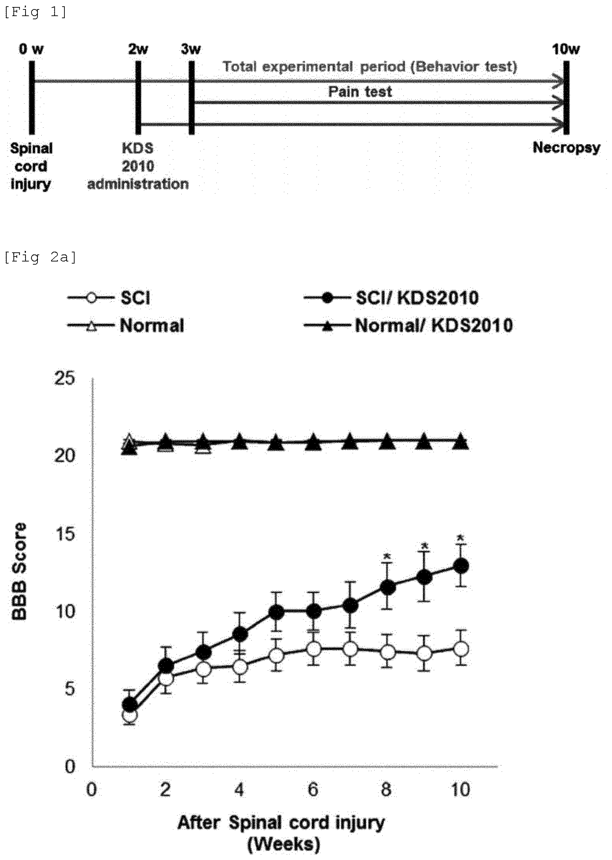

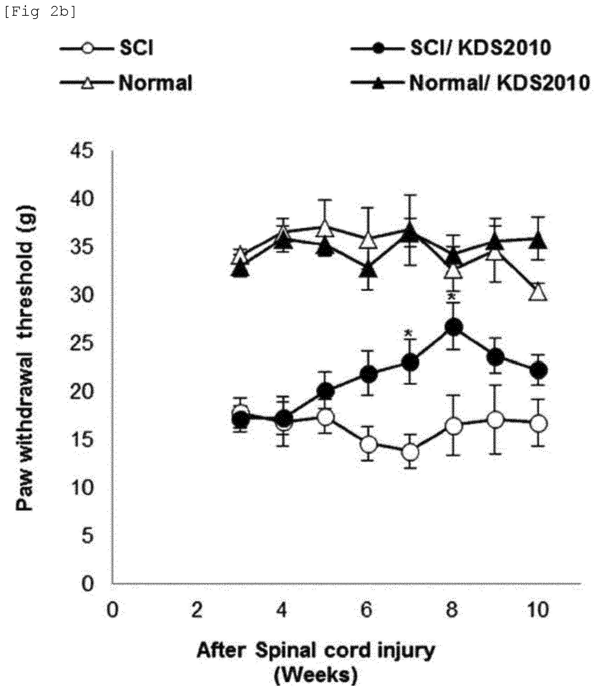

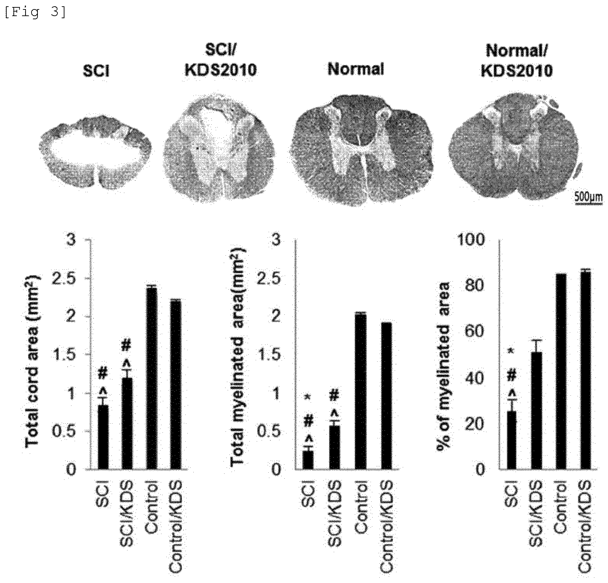

In a specific embodiment of the present invention, the result of treatment of an animal spinal cord injury model with the compound of Formula 1 of the present invention shows that nerve function recovery was facilitated, pain was relieved, and spinal cord tissue recovery was facilitated compared to a spinal cord-injured group not administered with the drug (FIGS. 2A, 2B and 3).

The result demonstrated that the composition containing the compound of Formula 1 of the present invention has an excellent effect of preventing or treating spinal cord injury.

As used herein, the term "preventing" or "prevention" refers to any action that inhibits or delays the onset of spinal cord injury by administration of the composition containing the compound of Formula 1 of the present invention. As used herein, the term "treatment" refers to any action which alleviates or beneficially alters the symptoms of the disease by administration of the composition containing the compound of Formula 1 of the present invention.

The pharmaceutical dosage form of the composition for preventing or treating spinal cord injury of the present invention may be used alone or bonded to or suitably combined with another pharmaceutically active compound.

The composition for preventing or treating spinal cord injury of the present invention may further include a pharmaceutically acceptable carrier.

The composition for preventing or treating spinal cord injury of the present invention can be prepared into a pharmaceutical formulation using a method well known in the art to provide rapid, sustained or delayed release of the active ingredient after administration to a mammal. In the preparation of the formulation, preferably, the active ingredient is mixed or diluted with a carrier or encapsulated into a carrier in the form of a container.

Accordingly, the composition for preventing or treating spinal cord injury of the present invention is used as an oral formulation, such as a powder, granule, tablet, capsule, suspension, emulsion, syrup or aerosol, or a formulation such as an external preparation, suppository or sterile injectable solution, according to a conventional method. The composition may further include a suitable carrier, excipient and diluent commonly used in the preparation of compositions.

For example, the carrier that can be included in the composition of the present invention includes, but is not limited to, lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil and the like. The preparation is carried out using an ordinary diluent or excipient such as a filler, extender, binder, wetting agent, disintegrating agent or surfactant.

Solid formulations for oral administration include tablets, pills, powders, granules, capsules and the like, and the solid formulation is prepared by mixing at least one excipient such as starch, calcium carbonate, sucrose, lactose or gelatin. In addition, lubricants such as magnesium stearate and talc may also be used, in addition to simple excipients.

Liquid formulations for oral administration include suspensions, liquids/solutions, emulsions, syrups and the like. In addition to commonly used simple diluents, water and liquid paraffin, various excipients, such as wetting agents, sweeteners, fragrances, and preservatives, may be also included.

Formulations for parenteral administration include sterile aqueous solutions, non-aqueous solvents, suspensions, emulsions, lyophilized preparations and suppositories. Non-aqueous solvents and suspensions may include propylene glycol, polyethylene glycol, vegetable oils such as olive oil, and injectable esters such as ethyl oleate. As a base for suppositories, Witepsol, macrogol, tween 61, cacao butter, laurin butter and glycerogelatin may be used.

The pharmaceutical composition of the present invention may be administered as a single therapeutic agent or in combination with another therapeutic agent, and may be administered sequentially or simultaneously with a conventional therapeutic agent. The pharmaceutical composition may be administered once or several times. Considering all of the factors described above, it is important to administer the composition in an amount capable of obtaining the maximum effect in a minimum amount without causing side effects, and such an amount can be easily determined by those skilled in the art.

As used herein, the term "administration" refers to introduction of the pharmaceutical composition of the present invention into a patient by any suitable method, and the route of administration of the composition of the present invention may be any one of a variety of oral or parenteral routes enabling the composition to reach the target tissue.

The method of administration of the pharmaceutical composition according to the present invention is not particularly limited, and may be any method commonly used in the art. As a non-limiting example of the administration method, the composition may be administered by an oral or parenteral administration method. The pharmaceutical composition according to the present invention can be prepared into various formulations according to a desired administration method.

The frequency of administration of the composition of the present invention is not particularly limited, but may be once a day or several times a day in a portionwise manner.

A typical daily dosage of the composition containing the compound of Formula 1 of the present invention as an active ingredient may be 1 to 1,000 mg/kg, specifically 11 to 100 mg/kg, and the composition may be administered once or several times in a portionwise manner.

In another aspect, the present invention is directed to a food composition for preventing or alleviating spinal cord injury containing the compound represented by Formula 1 as an active ingredient.

The food composition for preventing or alleviating spinal cord injury of the present invention includes formulations such as pills, powders, granules, acupuncture agents, tablets, capsules, or liquids, and the foods to which the composition of the present invention can be added include, for example, various foods, such as beverages, gum, teas, vitamin complexes and health supplement foods.

The food composition for preventing or alleviating spinal cord injury of the present invention may include, as an essential ingredient, the composition or an active component thereof or a physiologically acceptable salt thereof and there is no particular limitation as to other ingredients. Similar to common foods, the food composition may further include additional ingredients such as various herbal extracts, food supplement additives or natural carbohydrates.

As mentioned above, food supplement additives may also be added, and the food supplement additives include food additives commonly used in the art, for example, flavoring agents, aromas, coloring agents, fillers, stabilizers and the like.

Examples of the natural carbohydrate include conventional sugars including monosaccharides such as glucose and fructose, disaccharides such as maltose and sucrose and polysaccharides such as dextrin and cyclodextrin, and sugar alcohols such as xylitol, sorbitol and erythritol. In addition to the ingredients described above, natural flavoring agents (for example, rebaudioside A, glycyrrhizin or the like) and synthetic flavoring agents (saccharin, aspartame or the like) can be advantageously used as flavoring agents.

In addition to the ingredients described above, the food composition for preventing or alleviating spinal cord injury of the present invention may include a variety of nutrients, vitamins, minerals (electrolytes), flavoring agents such as synthetic flavoring agents and natural flavoring agents, coloring agents and fillers (such as cheese or chocolate), pectic acid and salts thereof, alginic acid and salts thereof, organic acids, protective colloidal thickeners, pH-adjusting agents, stabilizers, preservatives, glycerin, alcohol, carbonizing agents used in carbonated beverages, and the like. In addition, the food composition may include natural fruit juice and fruit flesh for the production of fruit juice beverages and vegetable beverages. These ingredients can be used alone or in combination.

In the present invention, the health supplement food includes a health functional food, a health food and the like.

The term "health functional food" has the same meaning as food for special health use (FoSHU), and means a food having excellent pharmaceutical and medicinal effects, which is processed to efficiently provide the functions of bioregulation and nutrition supply. Here, "function" (or "functional") means obtaining effects useful for health applications, such as nutrient control or physiological actions on the structures and functions of the human body. The food of the present invention can be produced by a method commonly used in the art, and can be produced by adding raw materials and ingredients conventionally added in the art. In addition, any formulation of the food may also be used without limitation, as long as it is acceptable as food. The food composition of the present invention can be prepared into various types of formulations and has the advantages of being free from side effects that may occur upon long-term administration of the drug because it contains food as a raw material, unlike general drugs. In addition, owing to excellent portability thereof, the food according to the present invention can be taken as a supplement to improve the effects of preventing or alleviating spinal cord injury.

As used herein, the term "subject" may mean any animal, including a human, who has or is likely to develop spinal cord injury. The animal may be a human or a mammal, such as a cow, horse, sheep, pig, goat, camel, antelope, dog or cat, which requires treatment of similar symptoms, but is not limited thereto.

### [Best mode]

Hereinafter, the present invention will be described in more detail with reference to examples and experimental examples. However, it will be obvious to those skilled in the art that these examples and experimental examples are provided only for illustration of the present invention and should not be construed as limiting the scope of the present invention.

### Example 1: Test method

### 1-1. Preparation of drug to be administered

A target drug was prepared to determine the therapeutic effect thereof for spinal cord injury. The drug to be administered was prepared as described in Korean Patent Registration No. 10-1746060. Specifically, (S)-2-(((4'-trifluoromethylbiphenyl-4-yl)methyl)amino)propanamide methanesulfonate, disclosed in Example 9 in accordance with the synthesis method in the Patent gazette (hereinafter, referred to as "KDS2010", was synthesized.

### 1-2. Production of animal model for spinal cord injury

The animal models of spinal cord injury herein used were adult male Sprague-Dawley rats weighing 180 to 200 g, and the rats were anesthetized by intraperitoneal injection of ketamine. Laminectomy was conducted on the T9 portion to expose the spinal cord, the spinal cord was compressed for 10 seconds using a dedicated forceps (self-closed forceps, Germany) to induce spinal cord injury, and then the muscles and skin were sutured.

### 1-3. Administration of KDS2010 beverage to spinal cord injury model and behavior and pain response tests

The laboratory animal management and research guidelines were conducted with the permission of Laboratory Animal Science, Medicine Department, Yonsei University in compliance with the guidelines of the association for assessment and accreditation of laboratory animal care (AAALAC).

Of the 50 spinal cord injury animal models in total, 5 were a non-drug-administered control group (Normal), 5 were a drug-administered control group (Normal/KDS2010), 20 were a non-drug-administered group subjected to spinal cord injury (SCI), and 20 were a drug-administered group subjected to spinal cord injury (SCI/KDS2010). 2 weeks after induction of spinal cord injury, a mixture of the drug and drinking water was administered at a dose of 10 mg/kg.

A motor function test was performed 10 times in total weekly for 10 weeks after spinal cord injury, and the recovery of hindlimb function in the animals was evaluated using the Basso-Beattie-Bresnahan (BBB) motor score. The BBB score is divided into 21 points in total, and is broadly classified into 3 steps. In the first recovery phase, the degree of the joint movement and whether or not the soles contact the floor was checked, in the second recovery phase, the degree of recovery of weighted gait was monitored, and in the final stage, the balance of the gait and the recovery of the tail were mainly observed. The neuropathic pain response test upon spinal cord injury was conducted through the measurement of a change in mechanical paw withdrawal using a dynamic plantar aesthesiometer and was conducted weekly for 3 weeks after the injury causing response to the soles. This test was conducted by applying a force of 0 to 50 g to the sole of the paw of the hindlimb using a needle with a diameter of 0.5 mm for 20 seconds. At this time, the force applied when the sole withdrawal occurred due to pain was measured.

### 1-4. Histological analysis

For EC staining (Eriochrome (Solochrome) cyanine staining), spinal cord tissue was obtained at 10 weeks after spinal cord injury, fixed with 4% paraformaldehyde, and dehydrated to perform freeze embedding. EC staining was performed on the injured area (thoracic vertebrae #9, T9) in 5 rats for the control group, 10 rats for the spinal cord-injured non-drug-administered group and 10 rats for the spinal cord-injured drug-administered group.

The EC staining is a staining method that can compare the areas of tissue and the myelinated part between groups because the myelinated part is stained in blue, whereas other nuclei, nerves, and non-myelinated parts appear in white.

### Example 2: Test results

### 2-1. Determination of neurological recovery effect using Basso-Beattie-Bresnahan (BBB) locomotor rating system

A behavior test was performed using a Basso-Beattie-Bresnahan (BBB) locomotor rating system after administration of KDS2010 to spinal-cord-injured rats and normal rats in the same manner as in Example 1-3 above. The result of the behavior test was expressed as a BBB score.

As a result, as can be seen in FIG. 2A, the non-drug-administered control group (Normal) and the drug-administered control group (Normal/KDS2010) exhibited a normal value of 21 points, and the spinal cord-injured non-drug-administered group (SCI) exhibited a BBB score of 10 or less due to non-weighted gaits. Meanwhile, spinal cord-injured drug-administered group (SCI/KDS2010) exhibited a score of 10 points or higher due to weighted gaits starting at 5 weeks. In the last week, SCI/KDS2010 exhibited a balanced gait and restored behavioral function. In addition, SCI/KDS2010 was found to have a significantly improved BBB score compared to the group not administered with the drug and subjected to spinal cord injury.

Therefore, the results described above demonstrated that administration of the KDS2010 drug to the spinal cord injury model can facilitate nerve function recovery in the animal model.

### 2-2. Determination of pain relief effect using dynamic-plantar-test-based pain response evaluation

A pain relief degree was measured using a pain response evaluation based on a dynamic plantar test after administration of KDS2010 to spinal-cord-injured rats and normal rats in the same manner as in Example 1-3 above.

As a result, as can be seen in FIG. 2B, the non-drug-administered control group (Normal) and the drug-administered control group (Normal/KDS2010) had a high threshold value (g) for pain, whereas a spinal cord-injured non-drug-administered group (SCI) had a low threshold value. On the other hand, the spinal cord-injured drug-administered group (SCI/KDS2010) was found to have a significantly increased threshold value for pain compared to the spinal cord-injured non-drug-administered group.

Therefore, the results described above demonstrated that administration of the KDS2010 drug to the spinal cord injury model can relieve pain in the animal model.

### 2-3. Determination of effect of facilitating recovery of spinal cord tissue

EC staining was conducted on the injured area (thoracic vertebrae #9, T9) of the non-drug-administered control group, the drug-administered control group, the spinal cord-injured non-drug-administered group and the spinal cord-injured drug-administered group, in the same manner as in Example 1-4, and the results of myelinated parts were quantified.

As a result, as can be seen in FIG. 3, the non-drug-administered control group (Control) and the drug-administered control group (Control/KDS2010) were large in all of total tissue area, total myelinated area, and the proportion (%) of the total myelinated area to the total tissue area, and the spinal cord-injured non-drug-administered group (SCI) was low in all of total tissue area, total myelinated area, and the proportion (%) of total myelinated area to total tissue area. These results demonstrated that spinal cord injury decreases all of total tissue area and total myelinated area.

In contrast, the spinal cord-injured drug-administered group (SCI/KDS2010) was found to have increased total tissue area and total myelinated area compared to the spinal cord-injured non-drug-administered group.

Therefore, the results described above demonstrated that administration of the KDS2010 drug to the spinal cord injury model can facilitate the recovery of spinal cord tissue.

### 2-4. Determination of re-myelination effect of spinal cord injury site

An additional analysis was conducted in order to investigate the results obtained in Example 2-3 in more detail.

Specifically, the number of myelinated exons, the number of cavities and a g-ratio were measured through toluidine blue (TB) staining and TEM imaging.

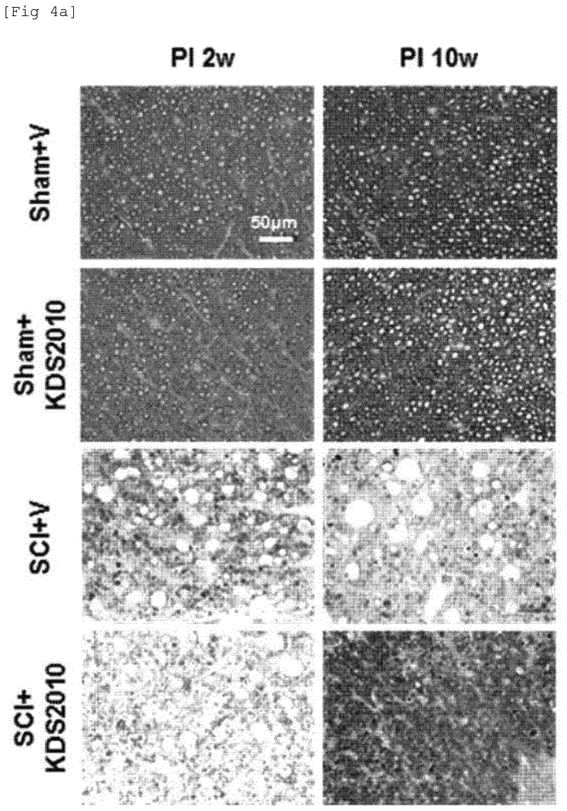

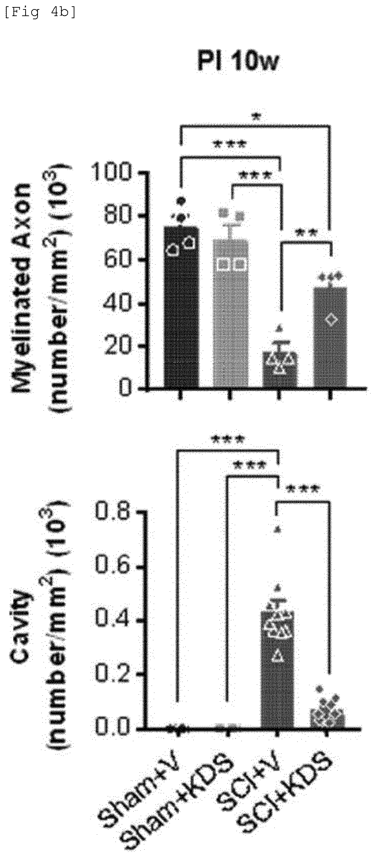

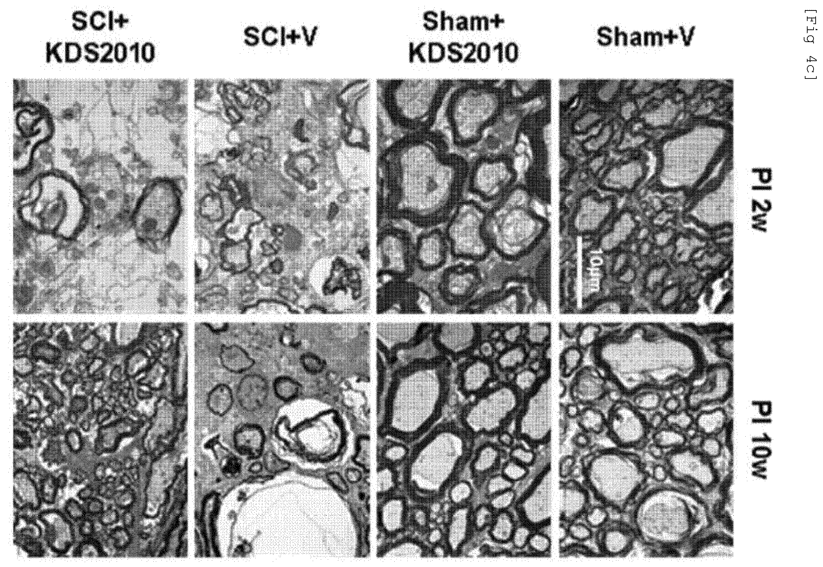

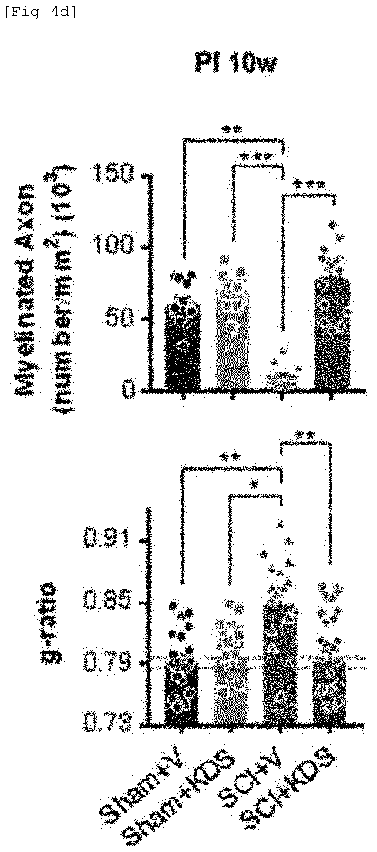

As a result, as can be seen from FIG. 4, the number of myelinated exons, which had decreased due to spinal cord injury, was recovered to an almost normal value by KDS2010 (FIG. 4A), whereas the number of cavities, which had significantly increased due to spinal cord injury, was rapidly decreased upon treatment with KDS2010 (FIG. 4B). In addition, the g-ratio, acting as an indicator of normal conductivity and velocity of recovered myelinated exons, was measured. The result showed that the spinal cord injury model had a g-ratio far different from the normal range of 0.79±0.0005, whereas KDS2010 had a g-ratio within the normal range.

Therefore, the results described above demonstrated that administration of the KDS2010 drug to the spinal cord injury model can facilitate re-myelination effect and thereby recover spinal cord tissue.

### 2-5. Determination of effect of spinal cord regeneration

A test to determine whether or not treatment with KDS2010 has an effect of inducing differentiation of new nerve cells was conducted in order to investigate the effect of spinal cord regeneration.

Specifically, BrdU was administered at a dose of 50 mg/kg/day to test animals by i.p. injection one week before scarification, and spinal nerves were subjected to immunofluorescence staining and then observed.

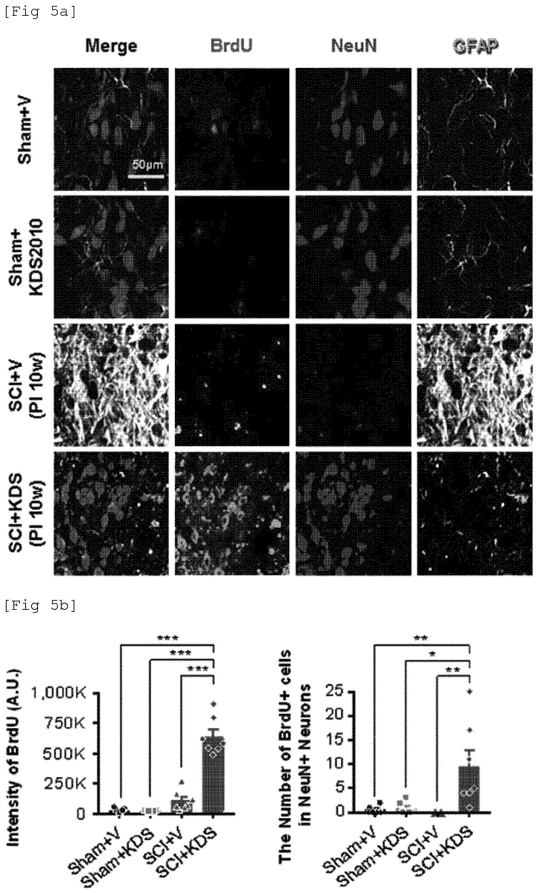

As a result, as can be seen from FIG. 5, the spinal cord injury model inhibited the formation of new nerve cells and led to the loss of nerve cells, whereas the animal model treated with KDS2010 exhibited newly formed nerve cells.

Therefore, the results described above demonstrated that the KDS2010 drug can facilitate the formation of spinal cord nerve cells and thus contribute to nerve generation upon spinal cord injury.

### 2-6. Determination of effect of inhibiting inflammatory reaction in spinal cord injury site

When a spinal cord injury occurs, destroyed cells and cell clusters were observed inward from the injured site. These cells may cause an inflammatory reaction and interfere with regeneration from spinal cord injury. A test was conducted to investigate the effect of the drug KDS2010 on inflammatory reaction.

Specifically, the spinal cord injury model administered with KDS2010 was subjected to immunofluorescence staining to determine whether or not the cells were positive for the macrophage marker CD68 and the microglia marker Iba1.

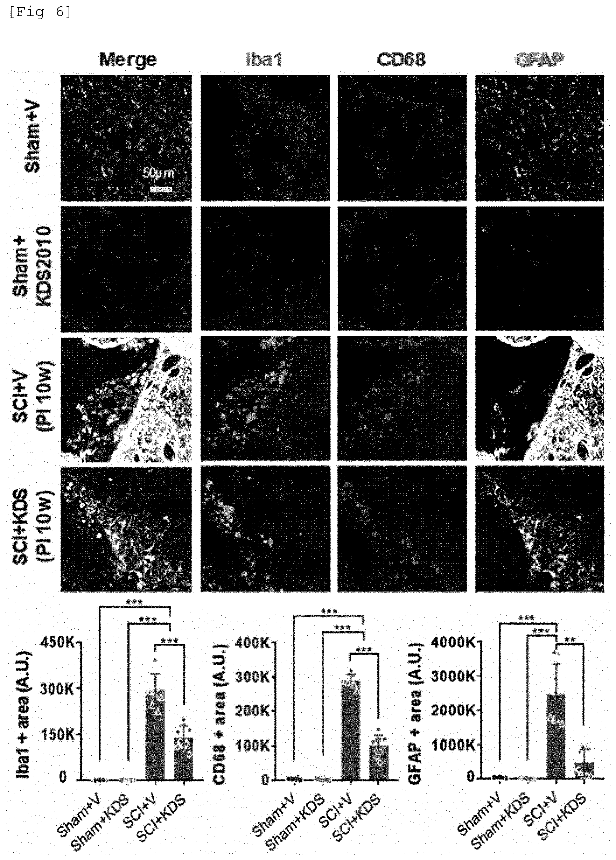

As a result, as can be seen from FIG. 6, the number of cells positive to CD68 and Iba1 significantly decreased in the KDS2010-administered spinal cord injury model group.

Therefore, the results described above demonstrated that administration with KDS2010 can reduce the number of inflammatory cells, which suppress spinal cord regeneration.

From the foregoing description above, those skilled in the art will appreciate that the present invention can be implemented in other specific embodiments without altering the technical idea or indispensable features thereof. In this regard, the embodiments described above are provided only for exemplary purposes, and should not be construed as limiting the scope of the present invention in all respects. Therefore, it should be interpreted that the meanings and scopes of the accompanying claims and all alterations or modifications derived from equivalents thereto, rather than the best mode, fall within the scope of the present invention.
thereto, rather than the best mode, fall within the scope of the present invention.

## Claims

1. A pharmaceutical composition for use in the prevention or treatment of spinal cord injury, comprising a compound represented by the following Formula 1 as an active ingredient:

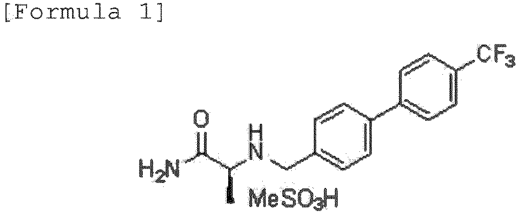

2. The pharmaceutical composition for use in the prevention or treatment of spinal cord injury according to claim 1, wherein the spinal cord injury is traumatic spinal cord injury or non-traumatic spinal cord injury.

3. A food composition for use in the prevention or alleviation of spinal cord injury comprising a compound represented by the following Formula 1 as an active ingredient:

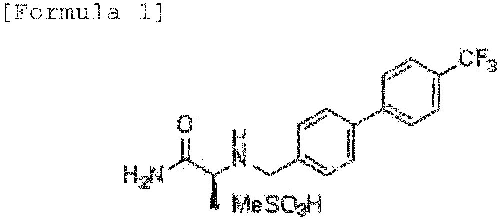

## Patentansprüche

1. - Pharmazeutische Zusammensetzung zur Verwendung bei der Vorbeugung oder Behandlung von Rückenmarksverletzung, umfassend eine Verbindung, die durch die folgende Formel 1 dargestellt ist, als aktiven Bestandteil:

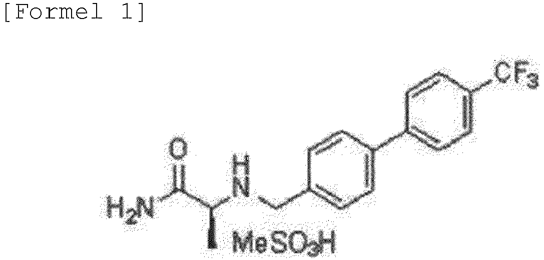

2. - Pharmazeutische Zusammensetzung zur Verwendung bei der Vorbeugung oder Behandlung von Rückenmarksverletzung nach Anspruch 1, wobei die Rückenmarksverletzung eine traumatische Rückenmarksverletzung oder eine nicht-traumatische Rückenmarksverletzung ist.

3. - Nahrungsmittelzusammensetzung zur Verwendung bei der Vorbeugung oder Linderung von Rückenmarksverletzung, umfassend eine Verbindung, die durch die folgende Formel 1 dargestellt ist, als aktiven Bestandteil:

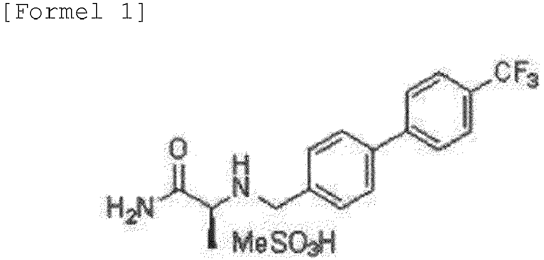

## Revendications

1. - Composition pharmaceutique destinée à être utilisée dans la prévention ou le traitement d'une lésion de la moelle épinière, comprenant un composé représenté par la formule 1 suivante en tant que principe actif :

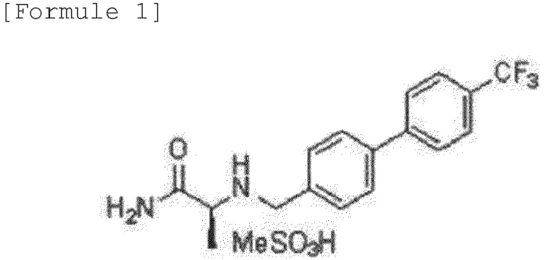

2. - Composition pharmaceutique destinée à être utilisée dans la prévention ou le traitement d'une lésion de la moelle épinière selon la revendication 1, dans laquelle la lésion de la moelle épinière est une lésion traumatique de la moelle épinière ou une lésion non traumatique de la moelle épinière.

3. - Composition alimentaire destinée à être utilisée dans la prévention ou le soulagement d'une lésion de la moelle épinière comprenant un composé représenté par la formule 1 suivante en tant que principe actif :
